# EUROPÄISCHE PATENTSCHRIFT

(11) **EP 0 503 436 B1**
(45) Veröffentlichungstag und Bekanntmachung des Hinweises auf die Patenterteilung: **16.10.1996**
(21) Anmeldenummer: 92103588.7
(22) Anmeldetag: 02.03.1992
(51) Int. Cl.: C07D 251/22, C07D 401/04, A01N 43/66, C07D 251/16, C07D 405/04

(54) **Triazinyl-substituierte Acrylsäureester**
Triazinyl substituted acrylic acid esters
Esters d'acide acrylique substitués par un groupement triazinyle

(30) Priorität: 13.03.1991 DE 4108029
(43) Veröffentlichungstag der Anmeldung: 16.09.1992
(73) Patentinhaber: BAYER AG, 51368 Leverkusen (DE)
(72) Erfinder: Knüppel, Peter C. Dr., W-5632 Wermelskirchen 3 (DE); Berg, Dieter, Professor. Dr., W-5600 Wuppertal (DE); Dutzmann, Stefan Dr., W-4010 Hilden (DE); Dehne, Heinz-Wilhelm, Dr. habil., W-4019 Monheim (DE); Hänssler, Gerd, Dr., W-5090 Leverkusen (DE)

(56) Entgegenhaltungen:
- EP-A- 0 178 826
- EP-A- 0 203 606
- EP-A- 0 212 859
- EP-A- 0 383 117
- EP-A- 0 409 369
- GB-A- 2 238 308

## Beschreibung

Die Erfindung betrifft neue triazinyl-substituierte Acrylsäureester, ein Verfahren zu ihrer Herstellung, ihre Verwendung in Schädlingsbekämpfungsmitteln und teilweise neue Zwischenprodukte.

Es ist bekannt, daß bestimmte substituierte Acrylsäureester, wie beispielsweise die Verbindung 3-Methoxy-2-(2-methylphenyl)-acrylsäuremethylester fungizide Eigenschaften besitzen (vgl. z.B. EP 178 826). Außerdem ist bekannt, daß bestimmte Pyrimidine, wie beispielsweise die Verbindung 2,4-Dichlor-5-methylthiopyrimidinyl-6-thiocyanat ebenfalls fungizide Eigenschaften besitzen (vgl. z.B. US 4.652.569: DE-OS 3 509 437 EP-A-383117, EP-A-212859).

Außerdem ist bekannt, daß Triazinyloxy(thio)acrylsäurederivate herbizide, fungizide und pflanzenwuchsregulatorische Wirkung zeigen (vergleiche EP-OS 409 369).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue triazinyl-substituierte Acrylsäureester der allgemeinen Formel (I) 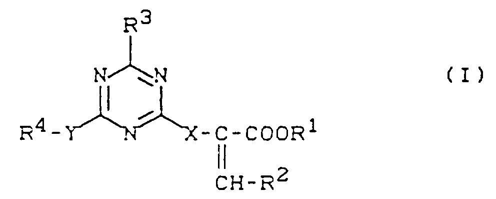 in welcher
- R¹: für geradkettiges oder verzweigtes Alkyl mit 1 bis 6 Kohlenstoffatomen steht,
- R²: für Dialkylamino mit jeweils 1 bis 6 Kohlenstoff-atomen in den einzelnen Alkylteilen steht oder für einen Rest -O-R⁵ steht,
- X: für Sauerstoff, Schwefel oder für einen Rest 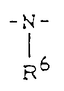 steht,
- R³: für Wasserstoff, C₁-C₆-Alkyl oder C₁-C₆-Alkoxy steht,
- R⁴: für jeweils gegebenenfalls einfach bis dreifach, gleich oder verschieden substituiertes Phenyl oder Pyridyl steht, wobei als Substituenten infrage kommen:
Fluor, Chlor, Brom, Methyl, Ethyl, t-Butyl, Cyclohexyl, Methoxy, Ethoxy, Trifluormethyl, Trifluormethoxy, Dioxymethylen, Difluordioxymethylen, Dioxyethylen, Tetrafluordioxyethylen oder jeweils gegebenenfalls einfach bis dreifach, gleich oder verschieden durch Fluor, Chlor, Methyl, Methoxy oder Trifluormethyl substituiertes Phenyl, Phenoxy oder Phenylethinyl
- R⁵: für Wasserstoff, geradkettiges oder verzweigtes Alkyl mit 1 bis 6 Kohlenstoffatomen oder für Aralkyl mit 1 bis 4 Kohlenstoffatomen im geradkettigen oder verzweigten Alkylteil und 6 bis 10 Kohlenstoffatomen im Arylteil steht,
- R⁶: für Wasserstoff, für geradkettiges oder verzweigtes Alkyl mit 1 bis 6 Kohlenstoffatomen oder für jeweils gegebenenfalls im Arylteil einfach oder mehrfach, gleich oder verschieden substituiertes Aralkyl oder Aryl mit 1 bis 6 Kohlenstoffatomen im geradkettigen oder verzweigten alkylteil und 6 bis 10 Kohlenstoffatomen im jeweiligen Arylteil steht, wobei als Substituenten im Arylteil jeweils die bei R⁴ genannten Substituenten infrage kommen und
- Y: für eine direkte Bindung, Sauerstoff oder für jeweils eine der folgenden Gruppierungen -CH₂- ; -CH=CH- oder -C≡C- steht,
gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen triazinyl-substituierten Acrylsäureester der allgemeinen Formel (I) 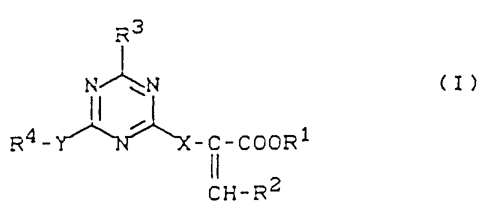 in welcher
R¹, R², R³, R⁴, X und Y die oben angegebenen Bedeutungen haben, deren Isomere oder Isomerengemische erhält, wenn man substituierte Essigsäureester der Formel (II) 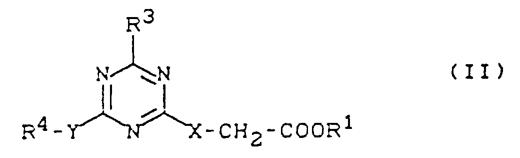 in welcher
R¹, R³, X, Y und R⁴ die oben angegebene Bedeutung haben, mit Alkoxy-bis-(dialkylamino)-methan-Verbindungen der Formel (III), 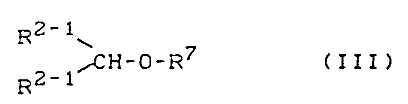 in welcher
- R²⁻¹: für Dialkylamino steht und
- R⁷: für Alkyl oder Cycloalkyl steht,
gegebenenfalls in Gegenwart eines Verdünnungsmittels umsetzt und gegebenenfalls anschließend in einer 2. Stufe die so erhältlichen 3-Dialkylaminoacrylsäurederivate der Formel (Ia), 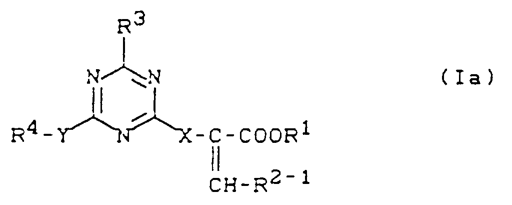 in welcher
- R¹, R²⁻¹, R³, R⁴, X und Y: die oben angegebene Bedeutung haben,
mit verdünnten Mineralsäuren hydrolysiert und anschließend in einer 3. Stufe die so erhältlichen 3-Hydroxyacrylsäureester der Formel (IV) 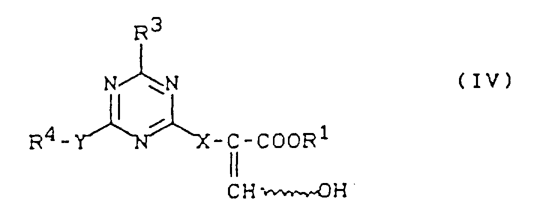 in welcher
R¹, R³, R⁴, X und Y die oben angegebene Bedeutung haben,
mit Alkylierungsmitteln der Formel (V),

R⁵-E¹ (V)

in welcher
- E¹: für eine elektronenanziehende Abgangsgruppe steht und
- R⁵: die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen triazinyl-substituierten Acrylsäureester der allgemeinen Formel (I) eine gute Wirkung gegen Schädlinge besitzen.

Überraschenderweise zeigen die erfindungsgemäßen triazinyl-substituierten Acrylsäureester der allgemeinen Formel (I) eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester oder die Verbindung 2,4-Dichlor-5-methylthiopyrimidinyl-6-thiocyanat, welche chemisch und wirkungsmäßig naheliegende Verbindungen sind.

Die erfindungsgemäßen triazinyl-substituierten Acrylsäureester sind durch die Formel (I) allgemein definiert. Bevorzugt sind Verbindungen der Formel (I), bei welchen Halogen, Alkyl, Cycloalkyl, Alkoxy, Alkylthio, Halogenalkyl, Halogenalkoxy, Halogenalkylthio, Dioxyalkylen, halogensubstituiertes Dioxyalkylen oder jeweils gegebenenfalls substituiertes Phenyl, Phenoxy, Benzyl, Phenylethyl, Phenylethenyl oder Phenylethinyl,
- R⁵: für Wasserstoff, geradkettiges oder verzweigtes Alkyl mit 1 bis 6 Kohlenstoffatomen oder für Aralkyl mit 1 bis 4 Kohlenstoffatomen im geradkettigen oder verzweigten Alkylteil und 6 bis 10 Kohlenstoffatomen im Arylteil steht,
- R⁶: für Wasserstoff, für geradkettiges oder verzweigtes Alkyl mit 1 bis 6 Kohlenstoffatomen oder für jeweils gegebenenfalls im Arylteil einfach oder mehrfach, gleich oder verschieden substituiertes Aralkyl oder Aryl mit 1 bis 6 Kohlenstoffatomen im geradkettigen oder verzweigten Alkylteil und 6 bis 10 Kohlenstoffatomen im jeweiligen Arylteil steht, wobei als Substituenten im Arylteil jeweils die bei R⁴ genannten Substituenten infrage kommen und
- Y: für eine direkte Bindung, Sauerstoff oder für jeweils eine der folgenden Gruppierungen -CH₂- ; -CH=CH- oder -C≡C- steht.

Besonders bevorzugt sind Verbindungen der Formel (I), bei welchen
- R¹: für Methyl, Ethyl, n- oder i-Propyl, n-, i-, s-oder t-Butyl steht,
- R²: für Dialkylamino mit jeweils 1 bis 4 Kohlenstoffatomen in den einzelnen Alkylteilen steht oder für einen Rest -O-R⁵ steht,
- X: für Sauerstoff, Schwefel oder für einen Rest 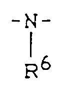 steht,
- R³: für Wasserstoff, C₁-C₄-Alkyl oder C₁-C₄-Alkoxy steht,
- R⁴: für jeweils gegebenenfalls einfach bis dreifach, gleich oder verschieden substituiertes Phenyl oder Pyridyl steht, wobei als Substituenten infrage kommen:
Fluor, Chlor, Brom, Methyl, Ethyl, t-Butyl, Cyclohexyl, Methoxy, Ethoxy, Trifluormethyl, Trifluormethoxy, Dioxymethylen, Difluordioxymethylen, Dioxyethylen, Tetrafluordioxyethylen oder jeweils gegebenenfalls einfach bis dreifach, gleich oder verschieden durch Fluor, Chlor, Methyl, Methoxy oder Trifluormethyl substituiertes Phenyl, Phenoxy oder Phenylethinyl,
- R⁵: für Wasserstoff, Methyl, Ethyl, n- oder i-Propyl, n-, i-, s-oder t-Butyl oder für Benzyl steht und
- R⁶: für Wasserstoff, Methyl, Ethyl, n- oder i-Propyl, n-, i-, s- oder t-Butyl oder für Benzyl oder Phenyl steht und
- Y: für eine direkte Bindung, Sauerstoff oder für jeweils eine der folgenden Gruppierungen -CH₂- ; -CH=CH- oder -C≡C- steht.

Ganz besonders bevorzugt sind Verbindungen der Formel (I), bei welchen
- R¹: für Methyl oder Ethyl steht,
- R²: für Dimethylamino, Hydroxy, Methoxy oder Ethoxy steht,
- X: für einen Rest 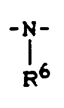 steht,
- R³: für Wasserstoff, C₁-C₄-Alkyl oder C₁-C₄-Alkoxy steht,
- R⁴: für jeweils gegebenenfalls einfach bis dreifach, gleich oder verschieden substituiertes Phenyl oder Pyridyl steht, wobei als Substituenten genannt seien:
Fluor, Chlor, Brom, Methyl, Ethyl, t-Butyl, Cyclohexyl, Methoxy, Ethoxy, Trifluormethyl, Trifluormethoxy, Dioxymethylen, Difluordioxymethylen, Dioxyethylen, Tetrafluordioxyethylen oder jeweils gegebenenfalls einfach bis dreifach, gleich oder verschieden durch Fluor, Chlor, Methyl, Methoxy oder Trifluormethyl substituiertes Phenyl, Phenoxy oder Phenylethinyl,
- R⁶: für Wasserstoff, Methyl, Ethyl oder Benzyl steht und
- Y: für eine direkte Bindung, Sauerstoff oder für jeweils eine der folgenden Gruppierungen -CH₂- -CH=CH- oder -C≡C- steht.

Die hier aufgeführten Definitionen der Reste gelten in entsprechender Weise auch für die Ausgangs- und Zwischenprodukte.

Desweiteren stellen die oben aufgeführten Kombinationen bevorzugte Kombinationen der einzelnen Reste dar. Die einzelnen Restedefinitionen können jedoch auch untereinander, also auch zwischen den jeweiligen Vorzugsbereichen der angegebenen Kombinationen beliebig ausgetauscht werden.

Aryl als solches oder in Zusammensetzungen bedeutet vorzugsweise Phenyl oder Naphthyl, insbesondere Phenyl.

Alle aliphatischen Reste als solche oder in Zusammensetzungen sind geradkettig oder verzweigt und enthalten vorzugsweise 1 bis 6, insbesondere 1 bis 4, ganz besonders 1 oder 2 Kohlenstoffatome.

Halogen steht, wenn nicht anders definiert, vorzugsweise für Fluor, Chlor, Brom oder Iod, insbesondere für Fluor, Chlor oder Brom.

Im einzelnen seien außer den bei den Herstellungsbeispielen genannten Verbindungen die folgenden triazinylsubstituierten Acrylsäureester der allgemeinen Formel (I) genannt:

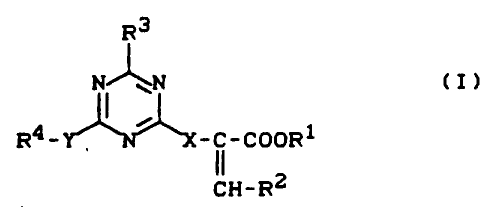

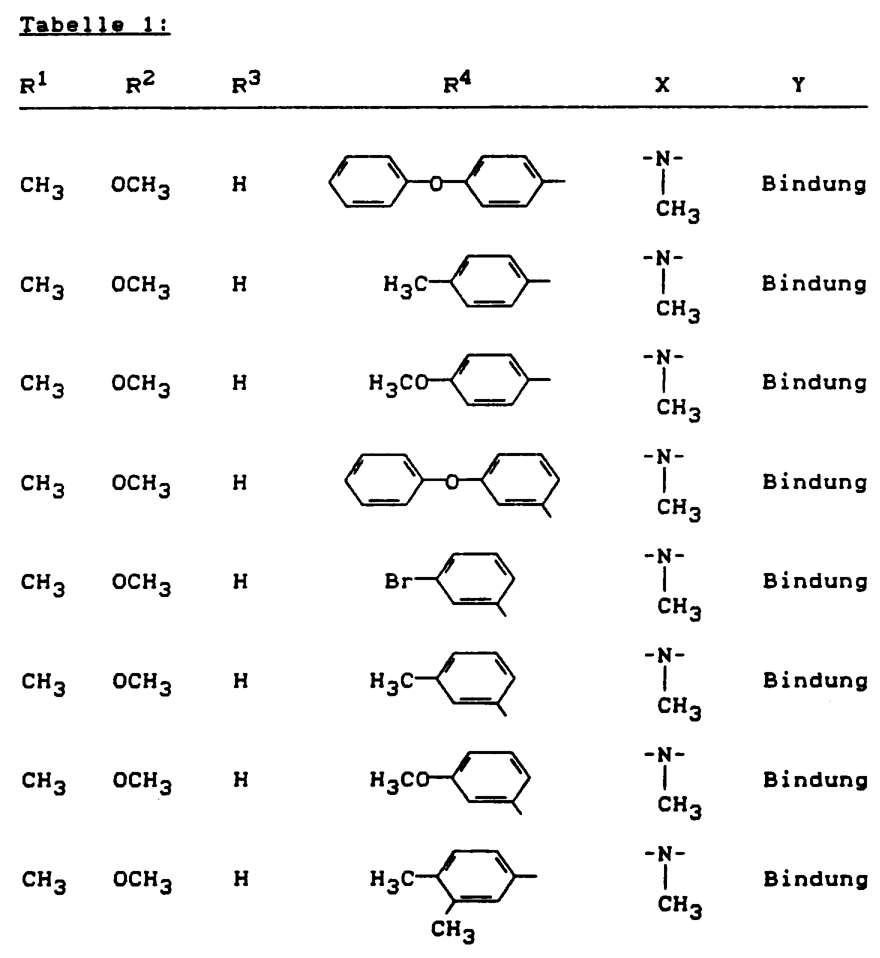

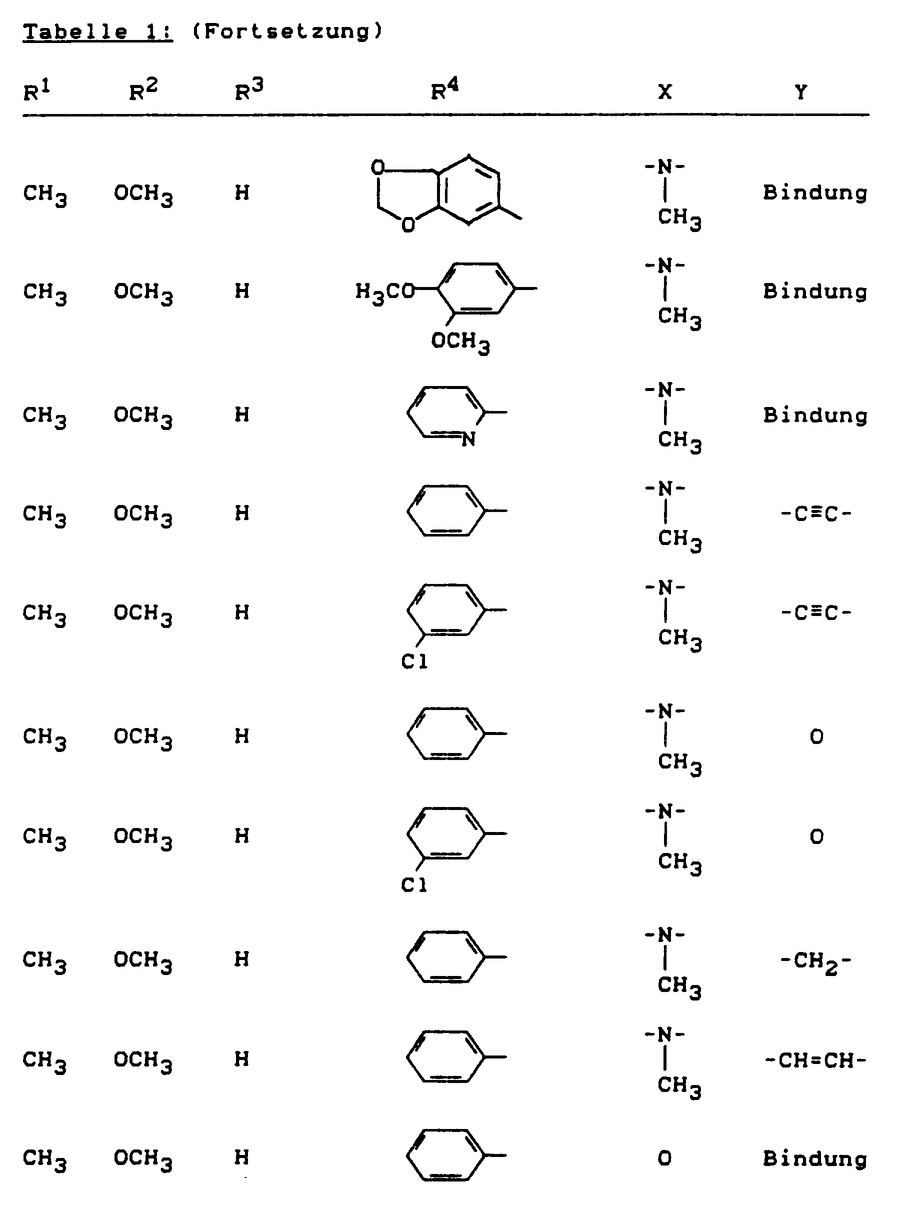

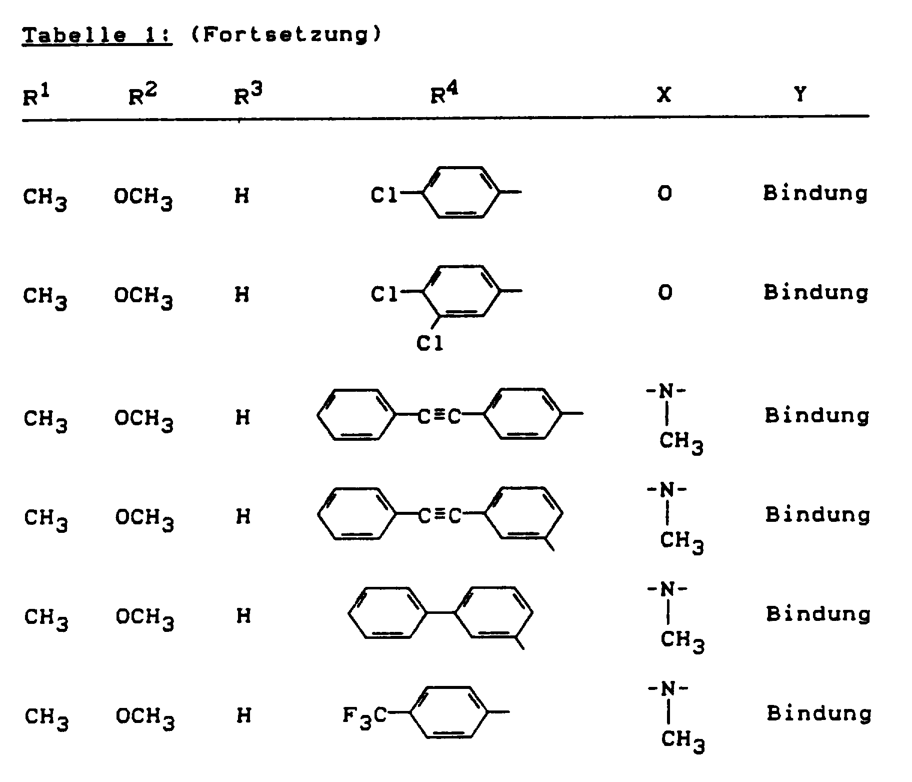

Verwendet man beispielsweise N-[2-(4-Phenyl)-triazinyl]--N-methylaminoessigsäuremethylester und t-Butoxy-bis(di-methylamino)-methan sowie Dimethylsulfat als Ausgangsstoffe, so läßt sich der Reaktionsablauf des erfindungsgemäßen Verfahrens durch das folgende Formelschema darstellen:

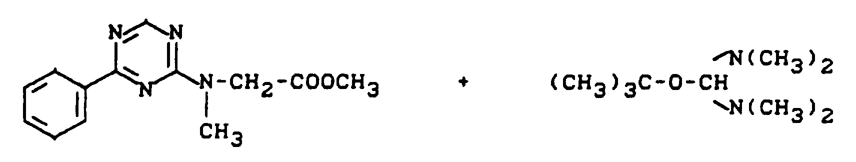

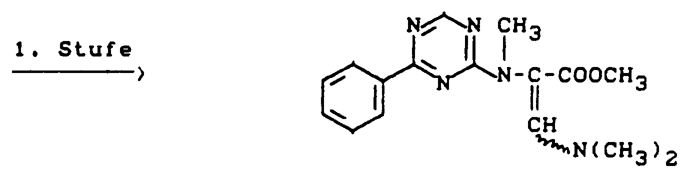

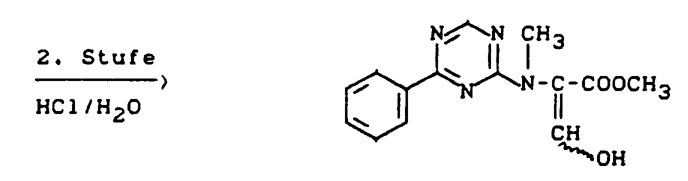

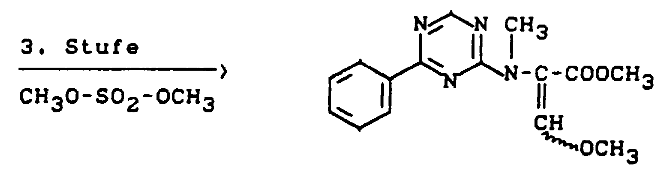

Die substituierten Essigsäureester der Formel (II) sind neu und Gegenstand der Erfindung. Sie können in Analogie zu literaturbekannten Methoden hergestellt werden, indem man halogensubstituierte 1,3,5-Triazinderivate der Formel (V) 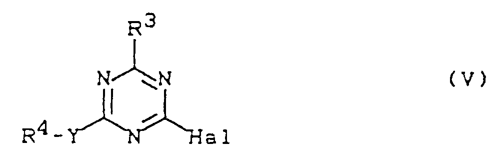 in welcher
- R³, R⁴ und Y: die oben angegebene Bedeutung haben und
- Hal: für Halogen, insbesondere Chlor steht,
mit Sarcosinsäureestern der Formel (VI) 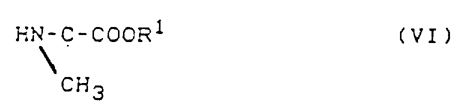 in welcher
- R¹: die oben angegebene Bedeutung hat,
gegebenenfalls in Gegenwart einer Base umgesetzt werden (vergleiche beispielsweise DE-OS 39 04 931 und Herstellungsbeispiel).

Die Verbindungen der Formel (V) sind bekannt (vergleiche z. B. WO 810 30 20; Aust. J. Chem., 34, 623 (1981); Synthesis 1980, 841).

Die Verbindungen der Formel (VI) sind allgemein bekannte Verbindungen der organischen Chemie.

Die zur Durchführung des erfindungsgemäßen Verfahrens weiterhin als Ausgangsstoffe benötigten Alkoxy-bis-(di-alkylamino)-methan-Verbindungen sind durch die Formel (III) allgemein definiert. In dieser Formel (III) steht R²⁻¹ vorzugsweise für Dialkylamino mit jeweils 1 bis 6 Kohlenstoffatomen, besonders bevorzugt 1 bis 4 Kohlenstoffatomen in den einzelnen geradkettigen oder verzweigten Alkylteilen.

R⁷ steht vorzugsweise für sekundäres oder tertiäres Alkyl mit 3 bis 8 Kohlenstoffatomen oder für Cycloalkyl mit 5 bis 7 Kohlenstoffatomen, insbesondere für t-Butyl oder Cyclohexyl.

Die Alkoxy-bis(dialkylamino)-methan-Derivate der Formel (III) sind bekannt (vgl. z.B. Chem. Ber. 101, 41-50 [1968]; Chem. Ber. 101, 1885-1888 [1968]; DE 23 03 919; PCT Int. Appl. WO 860 12 04) oder erhältlich in Analogie zu bekannten Verfahren. Die Zwischenprodukte der Formel (IV) sind neu und ebenfalls Gegenstand der Erfindung.

Die zur Durchführung des erfindungsgemäßen Verfahrens in der 3. Stufe weiterhin als Ausgangsstoffe benötigten Alkylierungsmittel sind durch die Formel (V) allgemein definiert. In dieser Formel (V) steht R⁵ vorzugsweise für diejenigen Reste, die bereits im Zusammenhang mit der Beschreibung der erfindungsgemäßen Stoffe der Formel (I) als bevorzugt für diesen Substituenten genannt wurden.
- E¹: steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (V) sind allgemein bekannte Verbindungen der organischen Chemie.

Als Verdünnungsmittel zur Durchführung der 1. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Amide wie Dimethylformamid, Dimethylacetamid, N-Methylformanilid, N-Methylpyrrolidon oder Hexamethylphosphorsäuretriamid.

Vorzugsweise wird die 1. Stufe des erfindungsgemäßen Verfahrens ganz ohne Zusatz von Lösungsmitteln durchzuführen.

Die Reaktionstemperaturen können bei der Durchführung der 1. Stufe des erfindungsgemäßen Verfahrens in einem größeren Bereich variiert werden. Im allgemeinen arbeitet man bei Temperaturen zwischen -35°C und +150°C, vorzugsweise bei Temperaturen zwischen 0°C und 120°C.

Die 1. Stufe des erfindungsgemäßen Verfahrens kann auch unter vermindertem oder erhöhtem Druck durchgeführt werden, bevorzugt arbeitet man jedoch unter Normaldruck.

Gegebenenfalls kann der Einsatz einer Inertgasatmosphäre wie beispielsweise Stickstoff oder Argon zweckmäßig sein, im allgemeinen ist es jedoch möglich, die 1. Stufe des erfindungsgemäßen Verfahrens unter normaler Raumluftatmosphäre durchzuführen.

Zur Durchführung der 1. Stufe des erfindungsgemäßen Verfahrens setzt man pro Mol an substituiertem Essigsäureester der Formel (II) im allgemeinen 1.0 bis 15.0 Mol, vorzugsweise 1.0 bis 12.0 Mol an Alkoxy-bis-(dialkylamino)-methan-Verbindung der Formel (III) ein.

Die Reaktionsdurchführung, Aufarbeitung und Isolierung der Reaktionsprodukte erfolgt nach allgemein üblichen Methoden (vgl. auch die Herstellungsbeispiele).

Als Säuren zur Durchführung der Hydrolyse in der 2. Stufe des erfindungsgemäßen Verfahrens kommen übliche anorganische und organische Säuren infrage. Vorzugsweise verwendet man wäßrige Lösungen von anorganischen Mineralsäuren, wie Salzsäure, Schwefelsäure, Phosphorsäure oder Salpetersäure; insbesondere wäßrige Salzsäure.

Als Verdünnungsmittel zur Durchführung der 2. und 3. Stufe des erfindungsgemäßen Verfahrens kommen ebenfalls inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die 2. Stufe des erfindungsgemäßen Verfahrens wird insbesondere in polaren Verdünnungsmitteln wie Acetonitril, Aceton oder Dimethylformamid gegebenenfalls auch in Gegenwart von Wasser durchgeführt.

Die 3. Stufe des erfindungsgemäßen Verfahrens kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-C₁₃/C₁₅-alkylammoniumchlorid, Dibenzyldimethyl-ammoniummethylsulfat, Dimethyl-C₁₂/C₁₄-alkylbenzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die 3. Stufe des erfindungsgemäßen Verfahrens wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 2. und 3. Stufe des erfindungsgemäßen Verfahrens in einem größeren Bereich variiert werden. Im allgemeinen arbeitet man bei Temperaturen zwischen -30°C und +150°C, vorzugsweise bei Temperaturen zwischen -20°C und +120°C.

Sowohl die 2. Stufe des erfindungsgemäßen Verfahrens als auch die 3. Stufe des erfindungsgemäßen Verfahrens werden im allgemeinen unter Normaldruck durchgeführt. Es ist jedoch auch möglich sie unter vermindertem oder erhöhtem Druck durchzuführen.

Zur Durchführung der 2. Stufe des erfindungsgemäßen Verfahrens setzt man pro Mol an 3-Dialkylaminoacrylsäureester der Formel (Ia) im allgemeinen 1.0 bis 3.0 Mol, vorzugsweise 1.0 bis 2.0 Mol an verdünnter Mineralsäure ein.
Die Reaktionsdurchführung, Aufarbeitung und Isolierung der Reaktionsprodukte erfolgt nach allgemein üblichen Methoden.

Zur Durchführung der 3. Stufe des erfindungsgemäßen Verfahrens setzt man pro Mol an 3-Hydroxyacrylsäureester der Formel (IV) im allgemeinen 1.0 bis 5.0 Mol, vorzugsweise 1.0 bis 3.0 Mol an Alkylierungsmittel der Formel (V) und gegebenenfalls 1.0 bis 5.0 Mol, vorzugsweise 1.0 bis 3.0 Mol an Reaktionshilfsmittel ein.
Die Reaktionsdurchführung, Aufarbeitung und Isolierung der Reaktionsprodukte erfolgt nach allgemein üblichen Methoden.

In einer besonders bevorzugten Variante führt man die 2. und die 3. Reaktionsstufe des erfindungsgemäßen Verfahrens in einer sogenannten "Eintopfreaktion" ohne Isolierung der Zwischenprodukte der Formel (IV) direkt in einem Reaktionsschritt durch. Auch bei dieser Variante erfolgt Reaktionsdurchführung, Aufarbeitung und Isolierung der Reaktionsprodukte nach allgemein üblichen Methoden (vgl. auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum; Phytophthora-Arten, wie beispielsweise Phytophthora infestans; Pseudoperonospora-Arten, wie beispielsweise Pseudoperonospora humuli oder Pseudoperonospora cubensis; Plasmopara-Arten, wie beispielsweise Plasmopara viticola; Peronospora-Arten, wie beispielsweise Peronospora pisi oder P. brassicae; Erysiphe-Arten, wie beispielsweise Erysiphe graminis; Sphaerotheca-Arten, wie beispielsweise Sphaerotheca fuliginea; Podosphaera-Arten, wie beispielsweise Podosphaera leucotricha; Venturia-Arten, wie beispielsweise Venturia inaequalis; Pyrenophora-Arten, wie beispielsweise Pyrenophora teres oder P. graminea (Konidienform: Drechslera, Syn: Helminthosporium); Cochliobolus-Arten, wie beispielsweise Cochliobolus sativus (Konidienform: Drechslera, Syn: Helminthosporium); Uromyces-Arten, wie beispielsweise Uromyces appendiculatus; Puccinia-Arten, wie beispielsweise Puccinia recondita; Tilletia-Arten, wie beispielsweise Tilletia caries; Ustilago-Arten, wie beispielsweise Ustilago nuda oder Ustilago avenae; Pellicularia-Arten, wie beispielsweise Pellicularia sasakii; Pyricularia-Arten, wie beispielsweise Pyricularia oryzae; Fusarium-Arten, wie beispielsweise Fusarium culmorum; Botrytis-Arten, wie beispielsweise Botrytis cinerea; Septoria-Arten, wie beispielsweise Septoria nodorum; Leptosphaeria-Arten, wie beispielsweise Leptosphaeria nodorum; Cercospora-Arten, wie beispielsweise Cercospora canescens; Alternaria-Arten, wie beispielsweise Alternaria brassicae; Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Getreidekrankheiten, wie beispielsweise gegen den Erreger des echten Getreidemehltaus an Gerste (Erysiphe graminis) oder gegen den Erreger der Braunfleckenkrankheit der Gerste (Cochliobolus sativus) oder gegen den Erreger des Apfelschorfs (Venturia inaequalis) oder zur Bekämpfung von Reiskrankheiten, wie beispielsweise gegen den Erreger der Reisfleckenkrankheit (Pyricularia oryzae) einsetzen. Hervorzuheben ist auch die gute in vitro-Wirksamkeit der erfindungsgemäßen Wirkstoffe. Auch die Zwischenprodukte der Formeln (II) und (IV) sind fungizid wirksam.

Außerdem zeigen die erfindungsgemäßen Verbindungen auch fungizide Wirkung gegen Leptosphaeria nodorum, Mehltau an Weizen, Pyrenophora teres und Fusarium nivale insbesondere an Getreide, gegen Phytophthora sowie eine blattinsektizide Nebenwirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die nachfolgenden Beispiele beschreiben die Herstellung und die Verwendung erfindungsgemäßer Wirkstoffe ohne sie darauf zu beschränken.

### Herstellungsbeispiele:

### Beispiel 1:

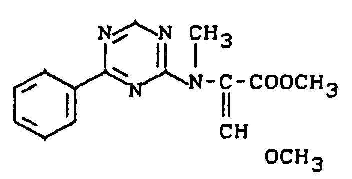

Zu 3,5 g (0.011 Mol) 2-{N-[4-phenyl-1,3,5-triazin-2-yl]-N-methylamino}-3-dimethylaminoacrylsäuremethylester in 350 ml Aceton gibt man zunächst 350 ml Wasser und anschließend 11,2 ml (0.022 Mol) 2normale Salzsäure und rührt anschließend 8 Stunden bei Raumtemperatur. Im Anschluß daran wird Aceton abdestilliert, die wäßrige Lösung neutralisiert und mit Essigester extrahiert. Die organische Phase wird eingeengt, der Rückstand in 100 ml Dimethylformamid aufgenommen, mit 2,5 g (0.021 Mol) Kaliumcarbonat und 3,2 g (0.026 Mol) Dimethylsulfat versetzt und 16 Stunden bei Raumtemperatur gerührt. Zur Aufarbeitung wird im Vakuum eingeengt, der Rückstand zwischen Essigester und Wasser verteilt. Die organische Phase wird eingeengt und der Rückstand an Kieselgel chromatographiert (Laufmittel: Essigester).

Man erhält 2.8 g (83% der Theorie) an 2-{N-[4-phenyl-1,3,5-triazin-2-yl]-N-methylamino}-3-methoxy-acrylsäuremethylester vom Schmelzpunkt 98°-101°C.

### Beispiel 2:

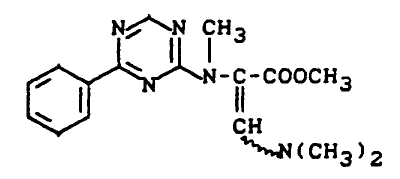

Zu 6,6 g (0.025 Mol) N-[4-phenyl-2-1,3,5-triazinyl]-N-methylglycinmethylester werden 50,0 g (0.286 Mol) t-Butyloxy-bis-(dimethylamino)methan gegeben. Nach 16stündigem Rühren bei 80°C wird das Reaktionsgemisch auf Wasser gegeben und mit Essigester extrahiert. Die vereinigten, getrockneten Essigesterphasen werden eingeengt.

Man erhält 6,5 g (83% der Theorie) an 2-{N-[4-phenyl-1,3,5-triazin-2-yl]-N-methylamino}-3-dimethylaminoacrylsäuremethylester als hellbraunes Öl.

In Analogie zu Beispiel 1 und 2 und unter Berücksichtigung der Angaben in der Beschreibung zu dem erfindungsgemäßen Verfahren, werden die nachfolgend in Tabelle 2 aufgeführten Endprodukte der Formel (I) 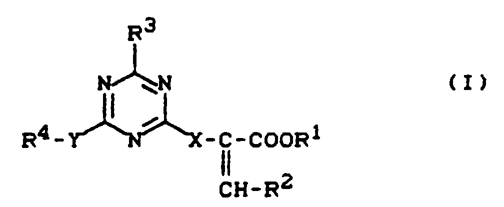 erhalten:

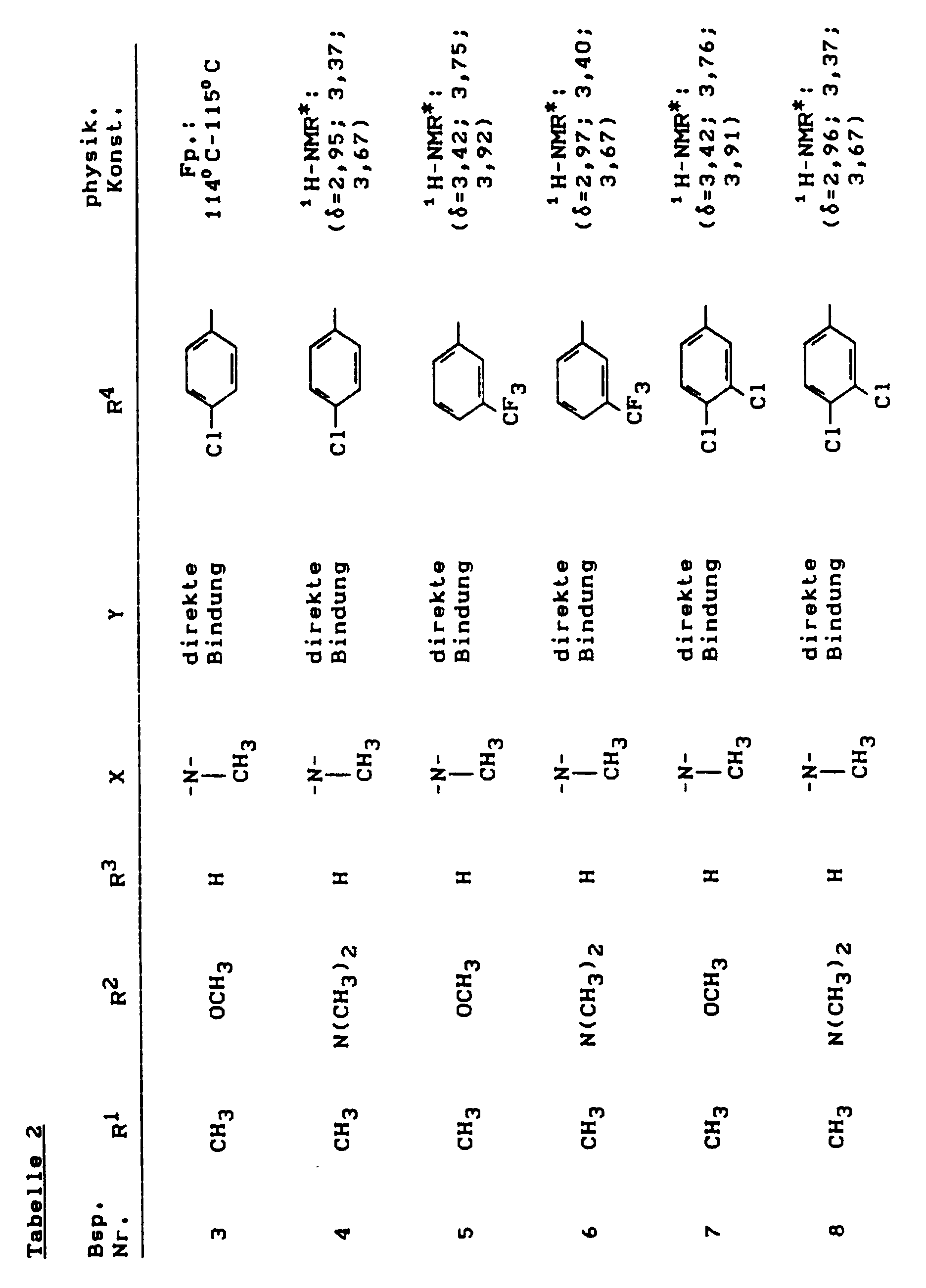

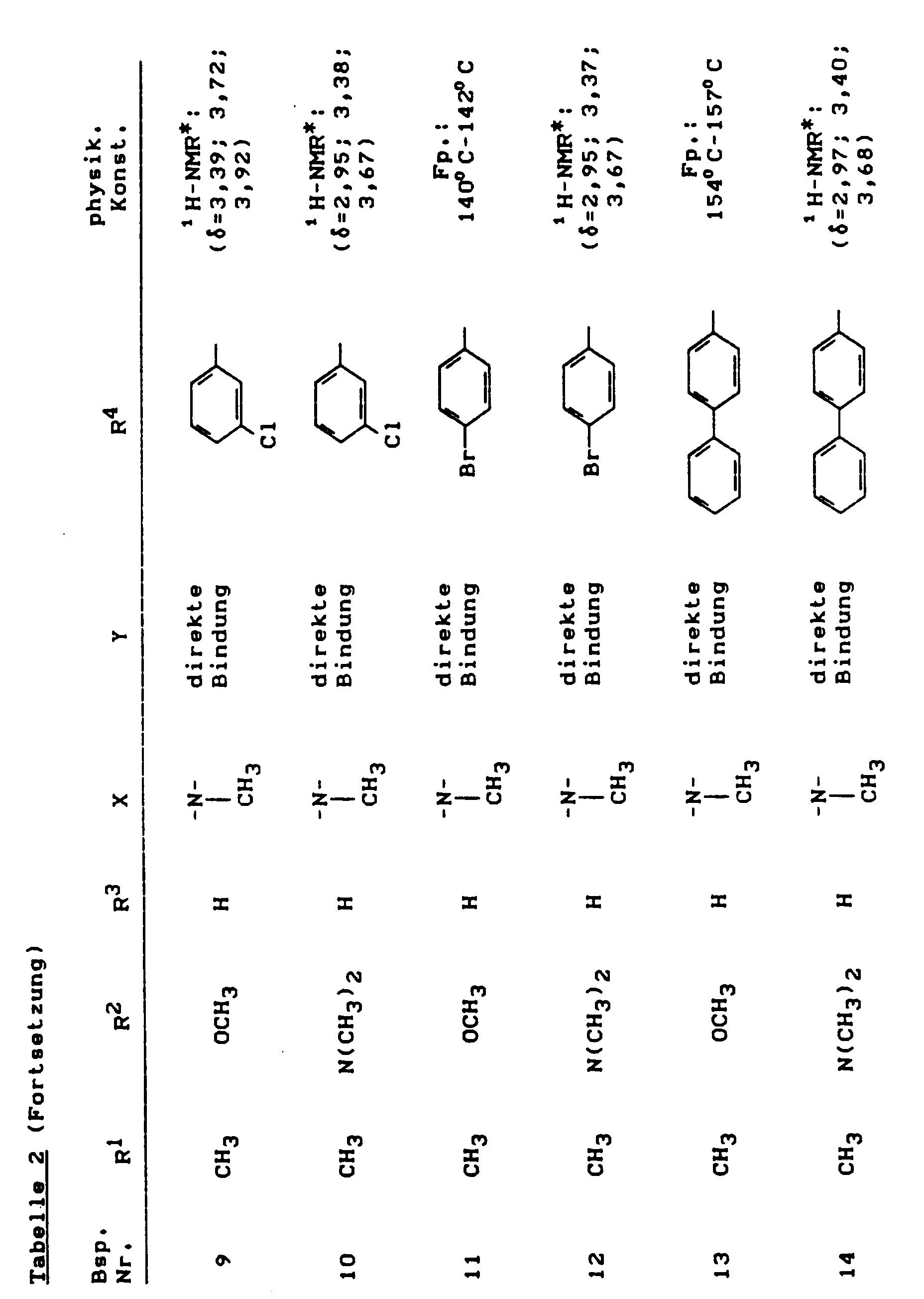

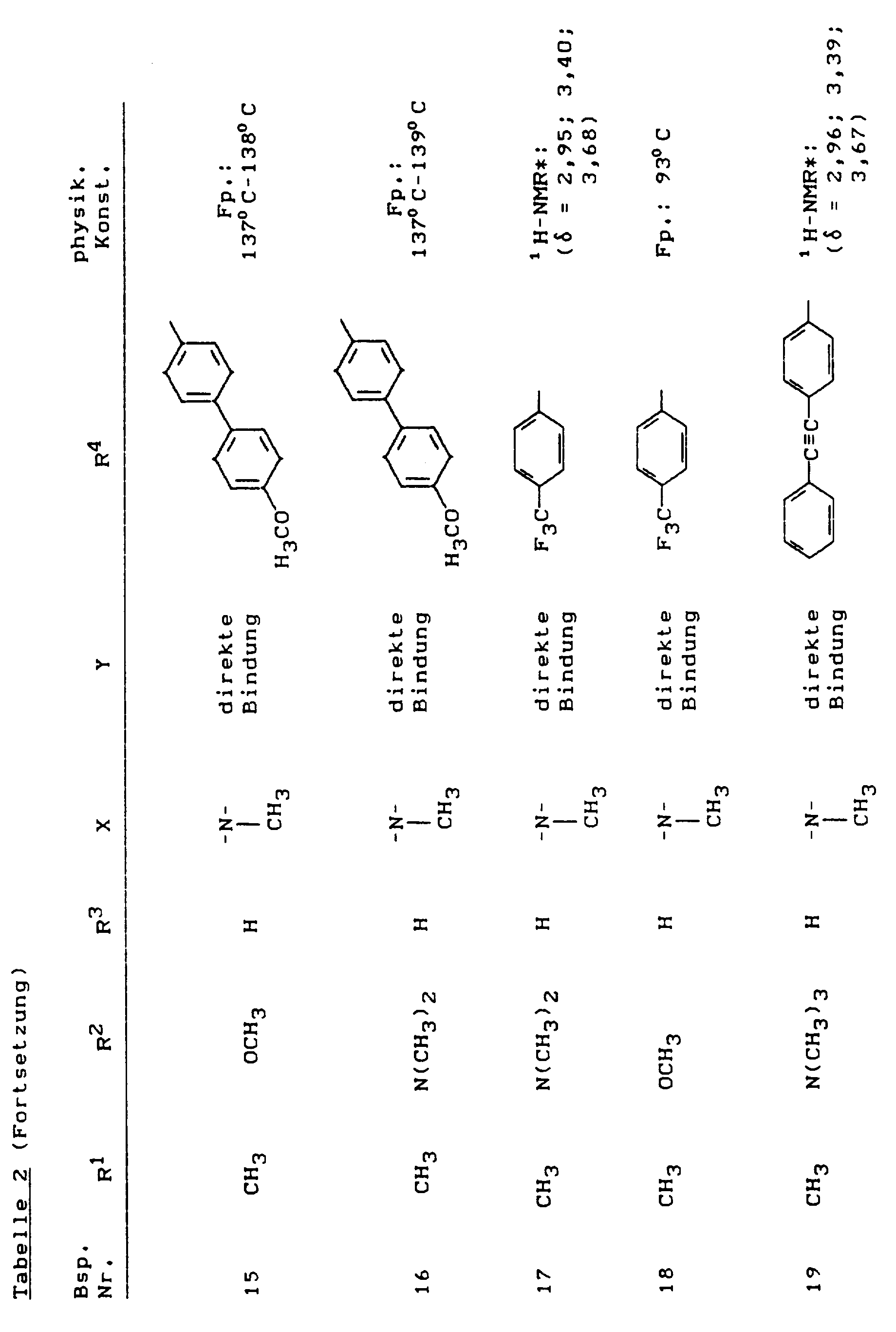

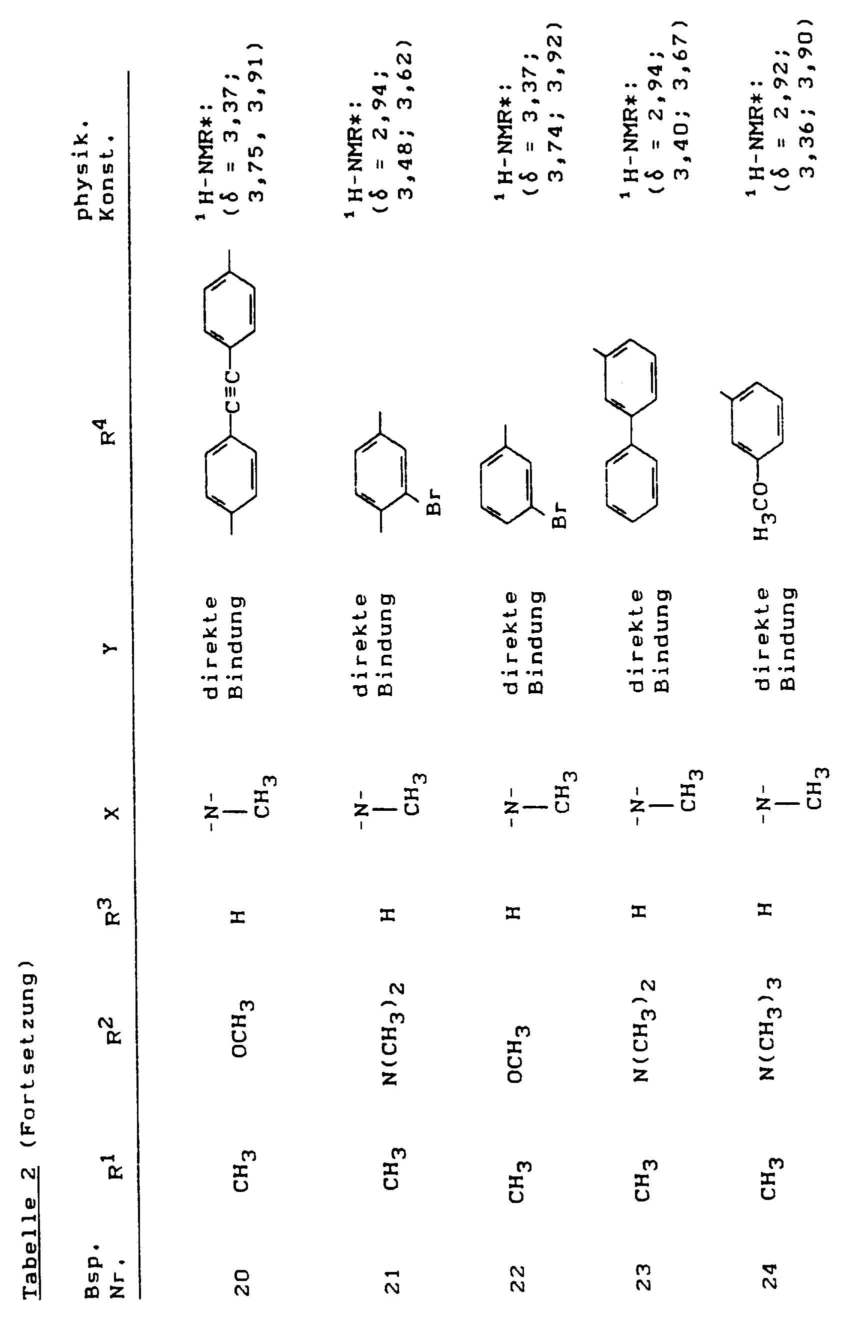

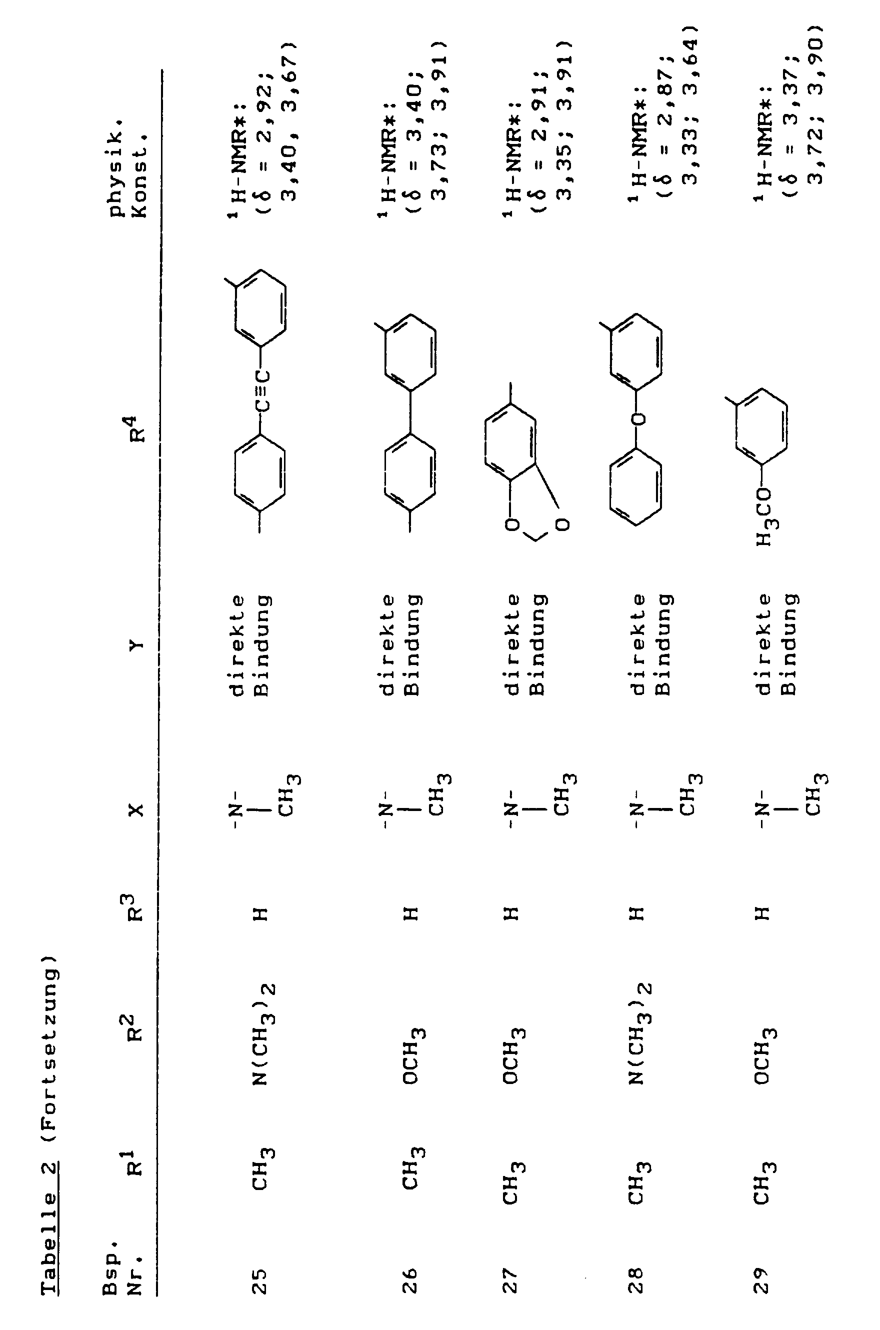

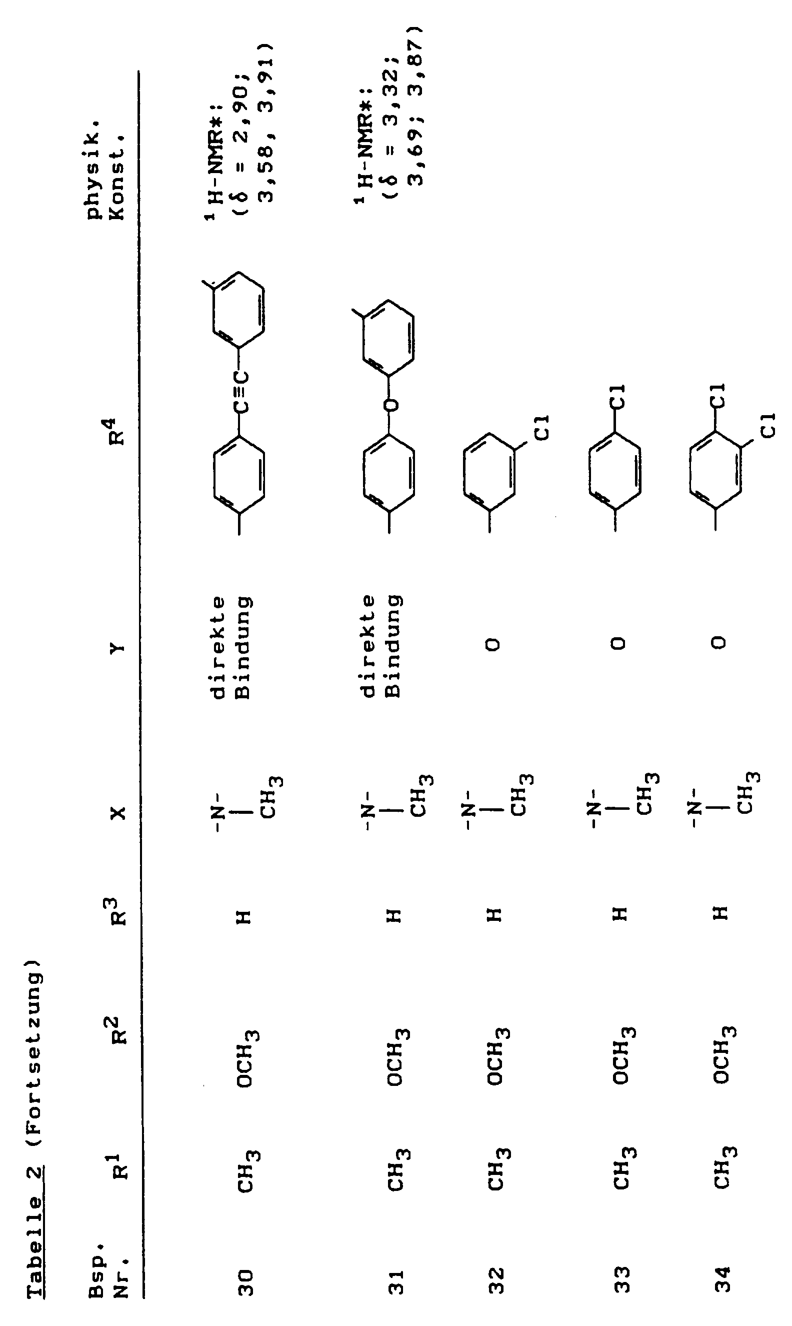

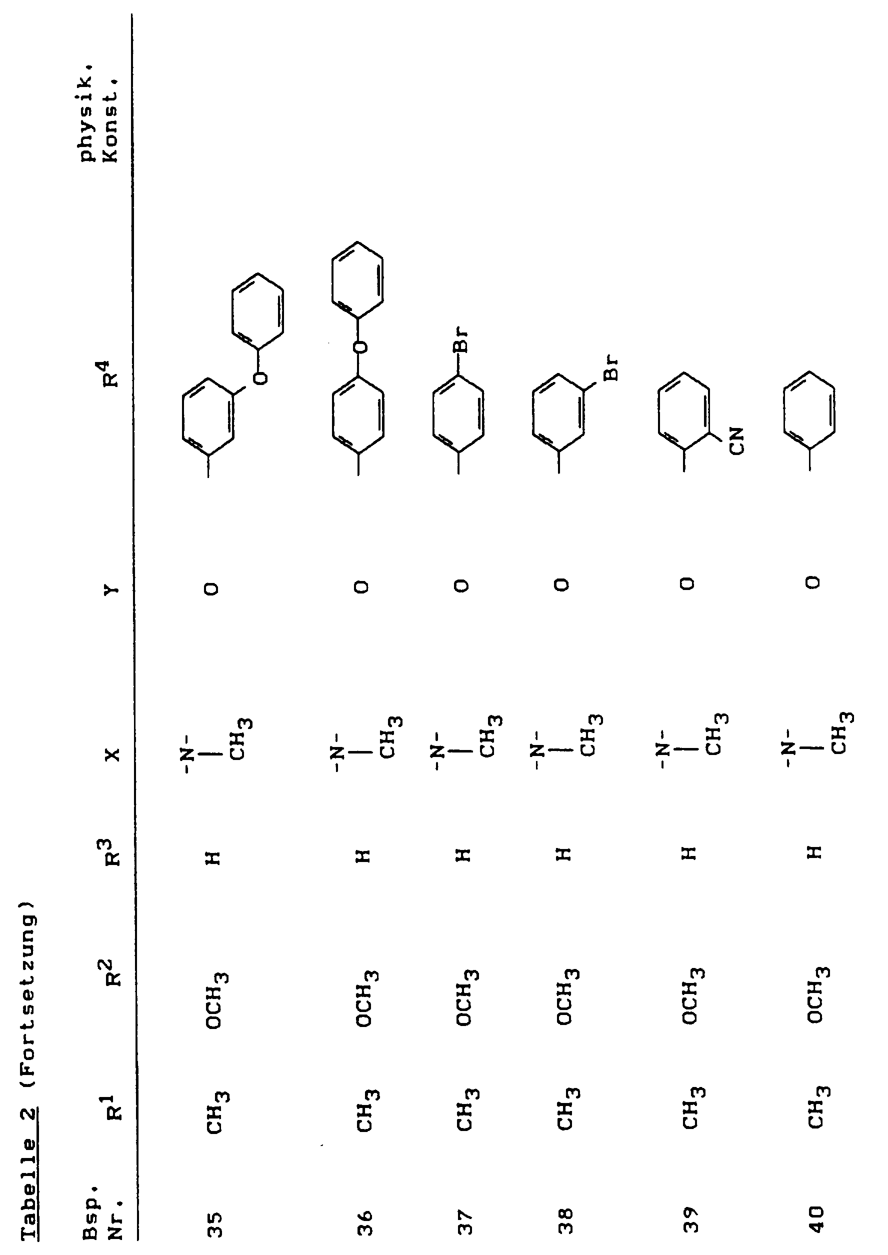

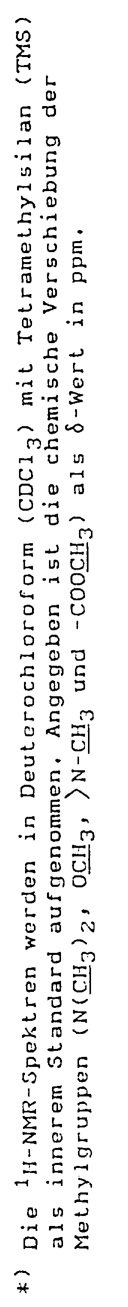

### Herstellung der Ausgangsverbindung:

### Beispiel II-1:

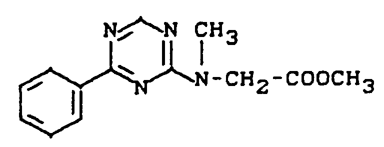

Zu 17,5 g (0.092 Mol) 2-Chlor-4-phenyl-1,3,5-triazin in 200 ml Dioxan werden 25,5 g (0.183 Mol) Sarcosinsäuremethylesterhydrochlorid und 27,7 g (0.274 Mol) Triethylamin gegeben und dieses Reaktionsgemisch 16 Stunden bei Raumtemperatur gerührt. Zur Aufarbeitung wird mit Wasser versetzt und mit Essigester extrahiert. Die vereinigten, getrockneten Essigesterphasen werden im Vakuum eingeengt und der Rückstand an Kieselgel chromatographiert (Laufmittel: Essigester, Hexan 1:3).

Man erhält 13,7 g (58% der Theorie) an N-[(phenyl-2-1,3,5-triazinyl)-N-methyl]-glycinmethylester vom Schmelzpunkt 71-73°C.

### Anwendungsbeispiele

In den folgenden Anwendungsbeispielen werden die nachstehend aufgeführten Verbindungen als Vergleichssubstanzen eingesetzt: 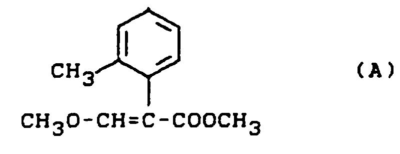 3-Methoxy-2-(2-methylphenyl)-acrylsäuremethylester (bekannt aus EP 178 826) 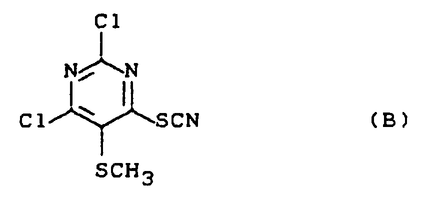 2,4-Dichlor-5-methylthiopyrimidinyl-6-thiocyanat (bekannt aus US-PS 4.652.569).

### Beispiel A

### Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine um mehr als 50 Wirkungsgrade höhere Wirksamkeit gegenüber dem Stand der Technik zeigen bei diesem Test z. B. die Verbindungen gemäß folgender Herstellungsbeispiele: 1 und 5.

### Beispiel B

### Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel 3.

### Beispiel C

### Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1, 7, 9, 11 und 13.

### Beispiel D

### Pyricularia-Test (Reis) / protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß folgender Herstellungsbeispiele: 1, 3 und 5.

## Patentansprüche

1. Acrylsäureester der allgemeinen Formel (I) 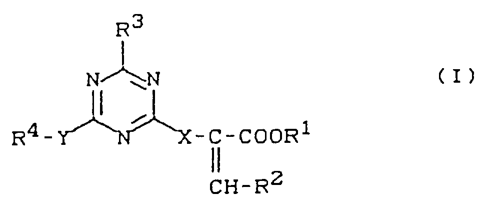 in welcher
R¹ für geradkettiges oder verzweigtes Alkyl mit 1 bis 6 Kohlenstoffatomen steht,
R² für Dialkylamino mit jeweils 1 bis 6 Kohlenstoffatomen in den einzelnen Alkylteilen steht oder für einen Rest -O-R⁵ steht,
X für Sauerstoff, Schwefel oder für einen Rest 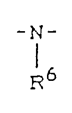 steht,
R³ für Wasserstoff, C₁-C₆-Alkyl oder C₁-C₆-Alkoxy steht,
R⁴ für jeweils gegebenenfalls einfach bis dreifach, gleich oder verschieden substituiertes Phenyl oder Pyridyl steht, wobei als Substituenten infrage kommen:
Fluor, Chlor, Brom, Methyl, Ethyl, t-Butyl, Cyclohexyl, Methoxy, Ethoxy, Trifluormethyl, Trifluormethoxy, Dioxymethylen, Difluordioxymethylen, Dioxyethylen, Tetrafluordioxyethylen oder jeweils gegebenenfalls einfach bis dreifach, gleich oder verschieden durch Fluor, Chlor, Methyl, Methoxy oder Trifluormethyl substituiertes Phenyl, Phenoxy oder Phenylethinyl,
R⁵ für Wasserstoff, geradkettiges oder verzweigtes Alkyl mit 1 bis 6 Kohlenstoffatomen oder für Aralkyl mit 1 bis 4 Kohlenstoffatomen im geradkettigen oder verzweigten Alkylteil und 6 bis 10 Kohlenstoffatomen im Arylteil steht,
R⁶ für Wasserstoff, für geradkettiges oder verzweigtes Alkyl mit 1 bis 6 Kohlenstoffatomen oder für jeweils gegebenenfalls im Arylteil einfach oder mehrfach, gleich oder verschieden substituiertes Aralkyl oder Aryl mit 1 bis 6 Kohlenstoffatomen im geradkettigen oder verzweigten Alkylteil und 6 bis 10 Kohlenstoffatomen im jeweiligen Arylteil steht, wobei als Substituenten im Arylteil jeweils die bei R⁴ genannten Substituenten infrage kommen und
Y für eine direkte Bindung, Sauerstoff oder für jeweils eine der folgenden Gruppierungen -CH₂- ; -CH=CH- oder -C≡C- steht.

2. Verbindungen der Formel (I) gemäß Anspruch 1, bei welchen
R¹ für Methyl, Ethyl, n- oder i-Propyl, n-, i-, s- oder t-Butyl steht,
R² für Dialkylamino mit jeweils 1 bis 4 Kohlenstoffatomen in den einzelnen Alkylteilen steht oder für einen Rest -O-R⁵ steht,
X für Sauerstoff, Schwefel oder für einen Rest 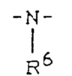 steht,
R³ für Wasserstoff, C₁-C₄-Alkyl oder C₁-C₄-Alkoxy steht,
R⁴ für jeweils gegebenenfalls einfach bis dreifach, gleich oder verschieden substituiertes Phenyl oder Pyridyl steht, wobei als Substituenten infrage kommen:
Fluor, Chlor, Brom, Methyl, Ethyl, t-Butyl, Cyclohexyl, Methoxy, Ethoxy, Trifluormethyl, Trifluormethoxy, Dioxymethylen, Difluordioxymethylen, Dioxyethylen, Tetrafluordioxyethylen oder jeweils gegebenenfalls einfach bis dreifach, gleich oder verschieden durch Fluor, Chlor, Methyl, Methoxy oder Trifluormethyl substituiertes Phenyl, Phenoxy oder Phenylethinyl, Dioxymethylen, Dioxyethylen, Tetrafluordioxyethylen, Difluordioxymethylen oder jeweils gegebenenfalls ein- bis dreifach, gleich oder verschieden durch Fluor, Chlor, Methyl, Methoxy oder Trifluormethyl substituiertes Phenyl, Phenoxy, Benzyl, Phenylethyl, Phenylethenyl oder Phenylethinyl,
R⁵ für Wasserstoff, Methyl, Ethyl, n- oder 1-Propyl, n-, i-, s- oder t-Butyl oder für Benzyl steht und
R⁶ für Wasserstoff, Methyl, Ethyl, n- oder 1-Propyl, n-, i-, s- oder t-Butyl oder für Benzyl oder Phenyl steht und
Y für eine direkte Bindung, Sauerstoff oder für jeweils eine der folgenden Gruppierungen -CH₂- ; -CH=CH- oder -C≡C- steht.

3. Verbindungen der Formel (I) gemäß Anspruch 1, bei welchen
R¹ für Methyl oder Ethyl steht,
R² für Dimethylamino, Hydroxy, Methoxy oder Ethoxy steht,
X für einen Rest 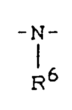 steht,
R³ für Wasserstoff, C₁-C₄-Alkyl oder C₁-C₄-Alkoxy steht,
R⁴ für jeweils gegebenenfalls einfach bis dreifach, gleich oder verschieden substituiertes Phenyl oder Pyridyl steht, wobei als Substituenten infrage kommen:
Fluor, Chlor, Brom, Methyl, Ethyl, t-Butyl, Cyclohexyl, Methoxy, Ethoxy, Trifluormethyl, Trifluormethoxy, Dioxymethylen, Difluordioxymethylen, Dioxyethylen, Tetrafluordioxyethylen oder jeweils gegebenenfalls einfach bis dreifach, gleich oder verschieden durch Fluor, Chlor, Methyl, Methoxy oder Trifluormethyl substituiertes Phenyl, Phenoxy oder Phenylethinyl,
R⁶ für Wasserstoff, Methyl, Ethyl oder Benzyl steht und
Y für eine direkte Bindung, Sauerstoff oder für jeweils eine der folgenden Gruppierungen -CH₂- -CH=CH- oder -C≡C- steht.

4. Verfahren zur Herstellung von Acrylsäureestern der allgemeinen Formel (I) 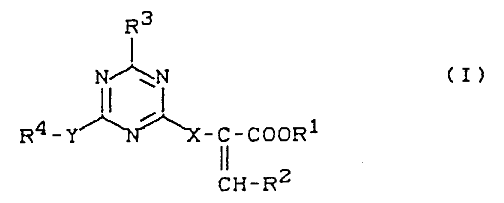 in welcher
R¹, R², R³, R⁴, X und Y die in Anspruch 1 angegebene Bedeutung haben,
dadurch gekennzeichnet, daß man substituierte Essigsäureester der Formel (II) 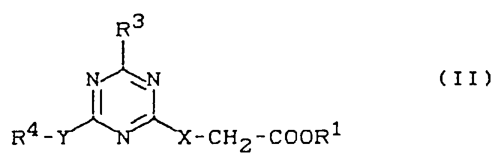 in welcher
R¹, R³, X, Y und R⁴ die oben angegebene Bedeutung haben,
mit Alkoxy-bis-(dialkylamino)-methan-Verbindungen der Formel (III), 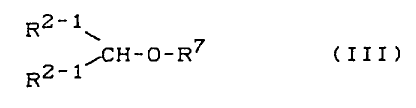 in welcher
R²⁻¹ für Dialkylamino steht und
R⁷ für Alkyl oder Cycloalkyl steht,
gegebenenfalls in Gegenwart eines Verdünnungsmittels umsetzt und gegebenenfalls anschließend in einer 2. Stufe die so erhältlichen 3-Dialkylaminoacrylsäurederivate der Formel (Ia), 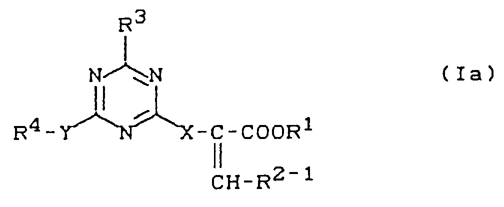 in welcher
R¹, R²⁻¹, R³, R⁴, X und Y die oben angegebene Bedeutung haben,
mit verdünnten Mineralsäuren hydrolysiert und anschließend in einer 3. Stufe die so erhältlichen 3-Hydroxyacrylsäureester der Formel (IV) 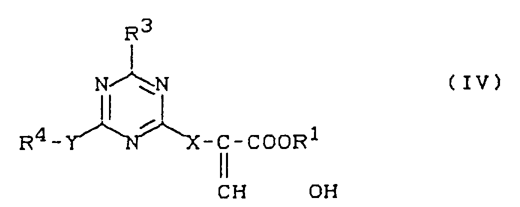 in welcher
R¹, R³, R⁴, X und Y die oben angegebene Bedeutung haben,
mit Alkylierungsmitteln der Formel (V),
R⁵-E¹ (V)
in welcher
E¹ für eine elektronenanziehende Abgangsgruppe steht und
R⁵ die oben angegebene Bedeutung hat,
gegebenenfalls in Gegenwart eines Verdünnungsmittels und gegebenenfalls in Gegenwart eines Reaktionshilfsmittels umsetzt.

5. Schädlingsbekämpfungsmittel, gekennzeichnet durch einen Gehalt an mindestens einem Acrylsäureester der Formel (I) nach den Ansprüchen 1 bis 4.

6. Verwendung von Acrylsäureestern der Formel (I) nach den Ansprüchen 1 bis 4 zur Bekämpfung von Schädlingen.

7. Verfahren zur Bekämpfung von Schädlingen, dadurch gekennzeichnet, daß man Acrylsäureester der Formel (I) nach den Ansprüchen 1 bis 4 auf Schädlinge und/oder ihren Lebensraum einwirken läßt.

8. Verfahren zur Herstellung von Schädlingsbekämpfungsmitteln, dadurch gekennzeichnet, daß man Acrylsäureester der Formel (I) nach den Ansprüchen 1 bis 4 mit Streckmitteln und/oder oberflächenaktiven Mitteln vermischt.

9. Essigsäureester der Formel (II) 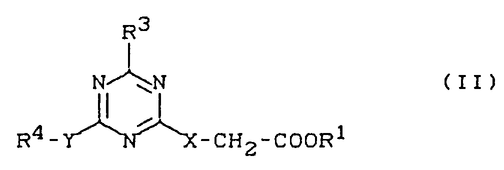 in welcher
R¹, R³, R⁴ und Y die in Anspruch 1 angegebene Bedeutung haben, und X für 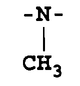 steht.

10. Verfahren zur Herstellung von Essigsäureestern der Formel (II), nach Anspruch 9 dadurch gekennzeichnet, daß halogensubstituierte 1,3,5-Triazinderivate der Formel (V) 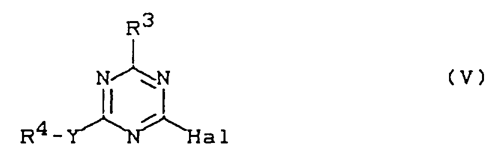 in welcher
R³, R⁴ und Y die oben angegebene Bedeutung haben und
Hal für Halogen steht,
mit Sarcosinsäureestern der Formel (VI) 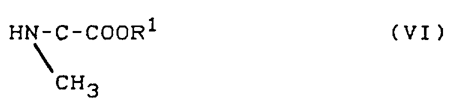 in welcher
R¹ die oben angegebene Bedeutung hat,
gegebenenfalls in Gegenwart einer Base umgesetzt werden.

## Claims

1. Acrylic esters of the general formula (I) 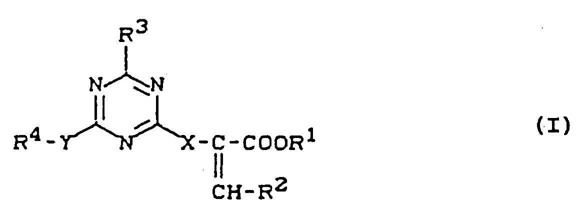 in which
R¹ represents straight-chain or branched alkyl having 1 to 6 carbon atoms,
R² represents dialkylamino having in each case 1 to 6 carbon atoms in the individual alkyl moieties, or represents a radical -O-R⁵,
X represents oxygen, sulphur or a radical 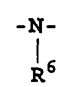
R³ represents hydrogen, C₁-C₆-alkyl or C₁-C₆-alkoxy,
R⁴ represents phenyl or pyridyl, each of which is optionally monosubstituted to trisubstituted by identical or different substituents, suitable substituents being: fluorine, chlorine, bromine, methyl, ethyl, t-butyl, cyclohexyl, methoxy, ethoxy, trifluoromethyl, trifluoromethoxy, dioxymethylene, difluorodioxymethylene, dioxyethylene, tetrafluorodioxyethylene, or phenyl, phenoxy or phenylethinyl, each of which is optionally monosubstituted to trisubstituted by identical or different substituents from the series comprising fluorine, chlorine, methyl, methoxy or trifluoromethyl,
R⁵ represents hydrogen, straight-chain or branched alkyl having 1 to 6 carbon atoms, or represents aralkyl which has 1 to 4 carbon atoms in the straight-chain or branched alkyl moeity and 6 to 10 carbon atoms in the aryl moeity,
R⁶ represents hydrogen, straight-chain or branched alkyl having 1 to 6 carbon atoms, or represents aralkyl or aryl which has 1 to 6 carbon atoms in the straight-chain or branched alkyl moiety and 6 to 10 carbon atoms in the respective aryl moiety, and each of which is optionally monosubstituted or polysubstituted in the aryl moiety by identical or different substituents, suitable substituents in the aryl moiety in each case being the substituents mentioned in the case of R⁴ and
Y represents a direct bond, oxygen, or in each case one of the following groups -CH₂-; -CH=CH-or -C≡C-.

2. Compounds of the formula (I) according to Claim 1, in which
R¹ represents methyl, ethyl, n- or i-propyl, n-, i-, s- or t-butyl,
R² represents dialkylamino having in each case 1 to 4 carbon atoms in the individual alkyl moieties, or represents a radical -O-R⁵,
X represents oxygen, sulphur or a radical 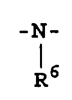
R³ represents hydrogen, C₁-C₄-alkyl or C₁-C₄-alkoxy,
R⁴ represents phenyl or pyridyl, each of which is optionally monosubstituted to trisubstituted by identical or different substituents, suitable substituents being: fluorine, chlorine, bromine, methyl, ethyl, t-butyl, cyclohexyl, methoxy, ethoxy, trifluoromethyl, trifluoromethoxy, dioxymethylene, difluorodioxymethylene, dioxyethylene, tetrafluorodioxyethylene, or phenyl, phenoxy or phenylethinyl, each of which is optionally monosubstituted to trisubstituted by identical or different substituents from the series comprising fluorine, chlorine, methyl, methoxy or trifluoromethyl,
R⁵ represents hydrogen, methyl, ethyl, n- or i-propyl, n-, i-, s- or t-butyl, or represents benzyl, and
R⁶ represents hydrogen, methyl, ethyl, n- or i-propyl, n-, i-, s- or t-butyl, or represents benzyl or phenyl, and
Y represents a direct bond, oxygen, or in each case one of the following groups -CH₂-; -CH=CH-or -C≡C-.

3. Compounds of the formula (I) according to Claim 1, in which
R¹ represents methyl or ethyl,
R² represents dimethylamino, hydroxyl, methoxy or ethoxy,
X represents a radical 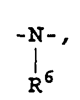
R³ represents hydrogen, C₁-C₄-alkyl or C₁-C₄-alkoxy,
R⁴ represents phenyl or pyridyl, each of which is optionally monosubstituted to trisubstituted by identical or different substituents, suitable substituents being: fluorine, chlorine, bromine, methyl, ethyl, t-butyl, cyclohexyl, methoxy, ethoxy, trifluoromethyl, trifluoromethoxy, dioxymethylene, difluorodioxymethylene, dioxyethylene, tetrafluorodioxyethylene or phenyl, phenoxy or phenylethinyl, each of which is optionally monosubstituted to trisubstituted by identical or different substituents from the series comprising fluorine, chlorine, methyl, methoxy or trifluoromethyl,
R⁶ represents hydrogen, methyl, ethyl or benzyl and
Y represents a direct bond, oxygen, or in each case one of the following groups -CH₂-; -CH=CH- or -C≡C-.

4. Process for the preparation of acrylic esters of the general formula (I) 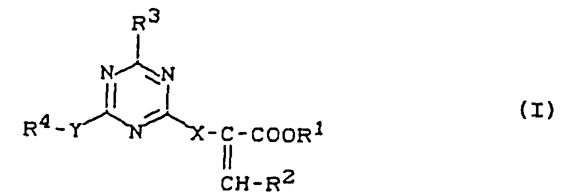 in which
R¹, R², R³, R⁴, X and Y have the meaning given in Claim 1,
characterised in that substituted acetic esters of the formula (II) 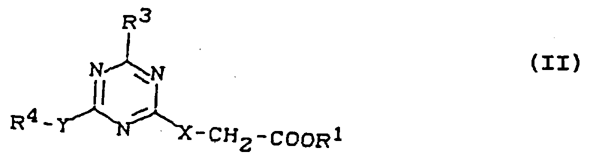 in which
R¹, R³, X, Y and R⁴ have the abovementioned meaning,
are reacted with alkoxy-bis-(dialkylamino)-methane compounds of the formula (III) 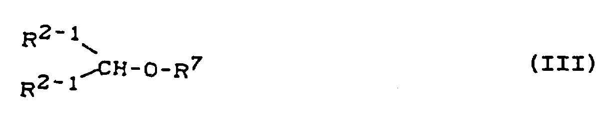 in which
R²⁻¹ represents dialkylamino and
R⁷ represents alkyl or cycloalkyl,
if appropriate in the presence of a diluent, and, if appropriate, the resulting 3-dialkylaminoacrylic acid derivatives of the formula (Ia) 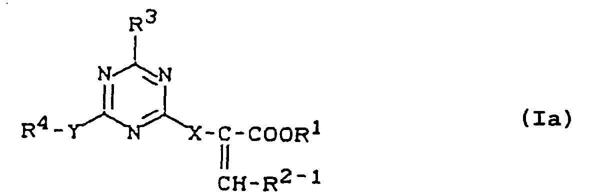 in which
R¹, R²⁻¹, R³, R⁴, X and Y have the abovementioned meaning,
are subsequently hydrolysed in a 2nd step using dilute mineral acids, and the resulting 3-hydroxyacrylic esters of the formula (IV) 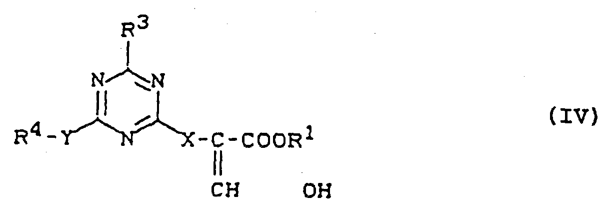 in which
R¹, R³, R⁴, X and Y have the abovementioned meaning,
are subsequently, in a 3rd step, reacted with alkylating agents of the formula (V)
R⁵-E¹ (V)
in which
E¹ represents an electron-withdrawing leaving group and
R⁵ has the abovementioned meaning,
if appropriate in the presence of a diluent and if appropriate in the presence of a reaction auxiliary.

5. Pesticides, characterised in that they contain at least one acrylic ester of the formula (I) according to Claims 1 to 4.

6. Use of acrylic esters of the formula (I) according to Claims 1 to 4 for combating pests.

7. Method of combating pests, characterised in that acrylic esters of the formula (I) according to Claims 1 to 4 are allowed to act on pests and/or their environment.

8. Process for the preparation of pesticides, characterised in that acrylic esters of the formula (I) according to Claims 1 to 4 are mixed with extenders and/or surface-active agents.

9. Acetic esters of the formula (II) 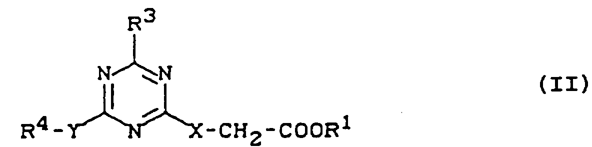 in which
R¹, R³, R⁴ and Y have the meaning given in Claim 1, and X represents

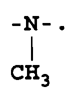

10. Process for the preparation of acetic esters of the formula (II) according to Claim 9, characterised in that halogen-substituted 1,3,5-triazine derivatives of the formula (V) 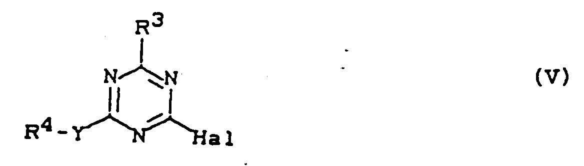 in which
R³, R⁴ and Y have the abovementioned meaning and
Hal represents halogen,
are reacted with sarcosic acid esters of the formula 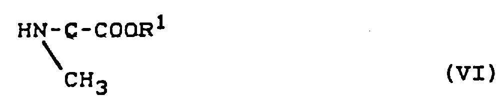 in which
R¹ has the abovementioned meaning,
if appropriate in the presence of a base.

## Revendications

1. Esters d'acide acrylique de la formule générale (I) 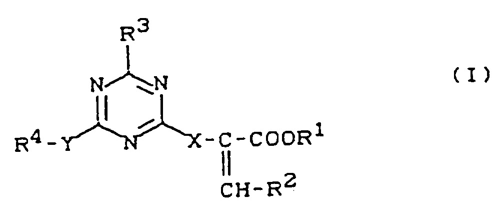 dans laquelle
R¹ représente un groupe alkyle ramifié ou linéaire avec 1 à 6 atomes de carbone,
R² représente un groupe dialkylamino avec respectivement 1 à 6 atomes de carbone dans les différentes parties d'alkyle ou un résidu -O-R⁵,
X représente l'oxygène, le soufre ou un résidu 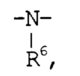
R³ représente l'hydrogène, un groupe alkyle en C₁-C₆ ou alkoxy en C₁-C₆,
R⁴ représente un groupe phényle ou pyridyle substitué respectivement le cas échéant de une à trois fois de manière identique ou différente, les substituants concernés étant les suivants :
fluor, chlore, brome, méthyle, éthyle, t-butyle, cyclohexyle, méthoxy, éthoxy, trifluorméthyle, trifluorméthoxy, dioxyméthylène, difluordioxyméthylène, dioxyéthylène, tétrafluordioxyéthylène ou phényle, phénoxy ou phényléthinyle substitué respectivement le cas échéant de une à trois fois, de manière identique ou différente par du fluor, du chlore, un groupe méthyle, méthoxy ou trifluorméthyle,
R⁵ représente l'hydrogène, un groupe alkyle linéaire ou ramifié avec 1 à 6 atomes de carbone ou un groupe aralkyle avec 1 à 4 atomes de carbone dans la partie d'alkyle linéaire ou ramifiée et 6 à 10 atomes de carbone dans la partie d'aryle,
R⁶ représente l'hydrogène, un groupe alkyle linéaire ou ramifié avec 1 à 6 atomes de carbone ou un groupe aralkyle ou aryle substitué respectivement le cas échéant de une à plusieurs fois de manière identique ou différente dans la partie d'aryle avec 1 à 6 atomes de carbone dans la partie d'alkyle ramifiée ou linéaire et 6 à 10 atomes de carbone dans la partie d'aryle respective, les substituants cités en R⁴ étant respectivement pris en compte en tant que substituants dans la partie d'aryle, et
Y représentant une liaison directe, l'oxygène ou l'un des groupes suivants respectivement -CH₂-; -CH=CH- ou -C=C-.

2. Composés de la formule (I) selon la revendication 1, dans lesquels
R¹ représente un groupe méthyle, éthyle, n-ou i-propyle, n-, i-, s- ou t-butyle,
R² représente un groupe dialkylamino avec chacun 1 à 4 atomes de carbone dans les différentes parties d'alkyle ou représente un résidu -O-R⁵,
X représente l'oxygène, le soufre ou un résidu 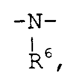
R³ représente l'hydrogène, un groupe alkyle en C₁-C₄ ou alkoxy en C₁-C₄,
R⁴ représente un groupe phényle ou pyridyle substitué respectivement le cas échéant de une à trois fois, de manière identique ou différente, les substituants concernés étant les suivants :
fluor, chlore, brome, méthyle, éthyle, t-butyle, cyclohexyle, méthoxy, éthoxy, trifluorméthyle, trifluorméthoxy, dioxyméthylène, difluordioxyméthylène, dioxyéthylène, tétrafluordioxyéthylène ou phényle, phénoxy ou phényléthinyle substitué respectivement le cas échéant de une à trois fois, de manière identique ou différente, par du fluor, du chlore, un groupe méthyle, méthoxy ou trifluorméthyle,
R⁵ représente l'hydrogène, un groupe méthyle, éthyle, n-ou i-propyle, n-, i-, s- ou t-butyle ou benzyle et
R⁶ représente l'hydrogène, un groupe méthyle, éthyle, n-ou i-propyle, n-, i-, s- ou t-butyle ou un groupe benzyle ou phényle et
Y représente une liaison directe, l'oxygène ou l'un des groupes suivants respectivement -CH₂-; -CH=CH- ou -C=C-.

3. Composés de la formule (I) selon la revendication 1, dans lesquels
R¹ représente un groupe méthyle ou éthyle,
R² représente un groupe diméthylamino, hydroxy, méthoxy ou éthoxy,
X représente un résidu 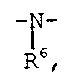
R³ représente l'hydrogène, un groupe alkyle en C₁-C₄ ou alkoxy en C₁-C₄,
R⁴ représente un groupe phényle ou pyridyle substitué respectivement le cas échéant de une à trois fois, de manière identique ou différente, les substituants concernés étant les suivants :
fluor, chlore, brome, méthyle, éthyle, t-butyle, cyclohexyle, méthoxy, éthoxy, trifluorméthyle, trifluorméthoxy, dioxyméthylène, difluordioxyméthylène, dioxyéthylène, tétrafluordioxyéthylène ou phényle, phénoxy ou phényléthinyle substitué respectivement le cas échéant de une à trois fois, de manière identique ou différente, par du fluor, du chlore, un groupe méthyle, méthoxy ou trifluorméthyle,
R⁶ représente l'hydrogène, un groupe méthyle, éthyle ou benzyle et
Y représente une liaison directe, l'oxygène ou l'un des groupes suivants respectivement -CH₂-; -CH=CH- ou -C=C-.

4. Procédé pour la fabrication d'esters d'acide acrylique de la formule générale (I) 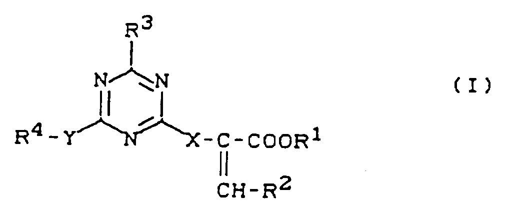 dans laquelle
R¹, R², R³, R⁴, X et Y présentent la signification indiquée à la revendication 1,
caractérisé en ce que l'on fait réagir des esters d'acide acétique substitués de la formule générale (II) 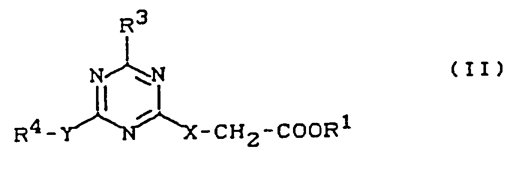 dans laquelle
R¹, R³, X, Y et R⁴ présentent la signification indiquée ci-dessus,
avec des composés alkoxy-bis-(dialkylamino)-méthane de la formule (III), 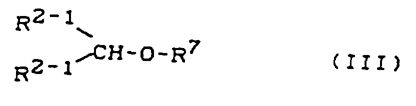 dans laquelle
R²⁻¹ représente un groupe dialkylamino et
R⁷ représente un groupe alkyle ou cycloalkyle,
éventuellement en présence d'un diluant et ensuite, éventuellement dans une seconde phase, on hydrolyse les dérivés d'acides 3-dialkylamino-acryliques de la formule (Ia) 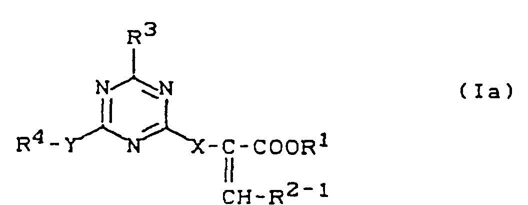 dans laquelle
R¹, R²⁻¹, R³, R⁴, X et Y présentent la signification indiquée ci-dessus,
avec des acides minéraux dilués et ensuite, dans une troisième phase, on fait réagir les 3-hydroxyacrylates de la formule (IV) 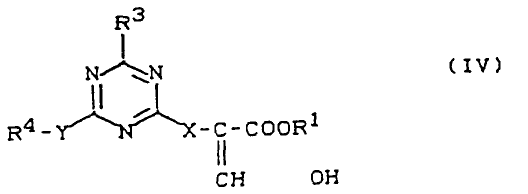 dans laquelle
R¹, R³, R⁴, X et Y présentent la signification indiquée ci-dessus,
avec des agents d'alkylation de la formule (V),
R⁵-E¹ (V)
dans laquelle
E¹ représente un groupe partant attirant les électrons et
R⁵ présente la signification indiquée ci-dessus,
éventuellement en présence d'un diluant et éventuellement en présence d'un auxiliaire de réaction.

5. Composition pour la lutte contre des parasites, caractérisée par une teneur en au moins un ester d'acide acrylique de la formule (I) selon les revendications 1 à 4.

6. Utilisation d'esters d'acide acrylique de la formule (I) selon les revendications 1 à 4 pour la lutte contre les parasites.

7. Procédé de lutte contre des parasites, caractérisé en ce qu'on fait agir l'ester d'acide acrylique de la formule (I) selon les revendications 1 à 4 sur les parasites et/ou sur leur milieu.

8. Procédé pour la fabrication de compositions pour la lutte contre des parasites, caractérisé en ce que l'on mélange l'ester d'acide acrylique de la formule (I) selon les revendications 1 à 4 à des diluants et/ou à des agents tensioactifs.

9. Esters d'acide acétique de la formule (II) 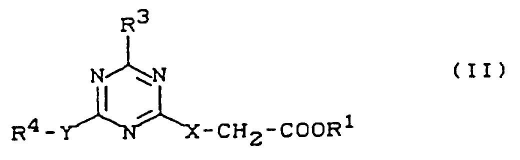 dans laquelle
R¹, R³, R⁴ et Y présentent la signification indiquée à la revendication 1 et X représente un groupe

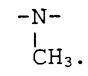

10. Procédé pour la fabrication d'esters d'acide acétique de la formule (II) selon la revendication 9, caractérisé en ce que des dérivés de 1,3,5-triazine substitués par des halogènes de la formule (V) 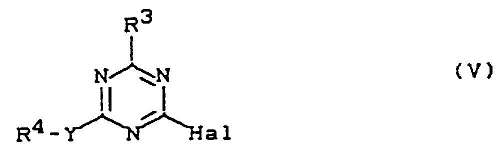 dans laquelle
R³, R⁴ et Y présentent la signification indiquée ci-dessus et
Hal représente un halogène,
sont amenés à réagir avec des esters d'acide sarcosique de la formule (VI) 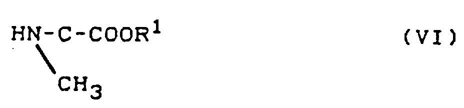 dans laquelle
R¹ présente la signification indiquée ci-dessus,
et ce, éventuellement en présence d'une base.
